# EUROPEAN PATENT SPECIFICATION

(11) **EP 3 784 141 B1**
(45) Date of publication and mention of the grant of the patent: **15.11.2023**
(21) Application number: 19718739.6
(22) Date of filing: 24.04.2019
(51) Int. Cl.: A61B 17/00

(54) **APPLICATOR FOR DEPOSITING A LAYER OF ADHESIVE OR SEALANT COMPOSITION ON A BIOLOGICAL AND/OR PROSTHETIC TISSUE**
APPLIKATOR ZUM AUFTRAGEN EINER SCHICHT EINES KLEBSTOFF- ODER DICHTSTOFFGEMISCHES AUF EIN BIOLOGISCHES UND/ODER PROTHETISCHES GEWEBE
APPLICATEUR POUR DÉPOSER UNE COUCHE DE COMPOSITION ADHÉSIVE OU D'ÉTANCHÉITÉ SUR UN TISSU BIOLOGIQUE ET/OU PROTHÉTIQUE

(30) Priority: 24.04.2018 EP 18305509
(43) Date of publication of application: 03.03.2021
(73) Proprietor: TISSIUM, 75012 Paris (FR)
(72) Inventor: LAMAZOUADE, Julien, 75011 Paris (FR); LOPES, Miguel, 92130 Issy les Moulineaux (FR); MENAND, Simon, 75012 Paris (FR); BIADILLAH, Youssef, 75014 Paris (FR); PEREIRA, Maria, 1200-366 Lisboa (PT)
(74) Representative: Regimbeau
(86) International application number: PCT/EP2019/060517
(87) International publication number: WO 2019/206998

(56) References cited:
- KR-B1- 101 666 865
- US-A- 4 946 079
- US-A- 6 063 055
- US-A1- 2005 127 119

## Description

### FIELD OF THE INVENTION

The invention relates to an applicator for depositing a layer of adhesive or sealant composition on a biological and/or prosthetic tissue, and to an applicator assembly including such an applicator.

### BACKGROUND ART

In surgical repair of vessels (such as aorta, femoral, carotid or coronary arteries, or veins), heart repair or urinary system repair, surgical adhesives or sealants are used as an adjunct to standard methods of achieving hemostasis (such as sutures and staples). Surgical adhesives or sealants may also be used for stopping leaks (for example brain or spinal cord leaks). Surgical adhesives/sealants are generally deposited as a layer over the bleeding or leaking site in order to seal the holes created by the sutures or the staples, to reinforce the junction between the tissue and facilitate hemostasis and tissue healing or filing the gaps.

However, the performance of adhesives/sealants does not only depend on the properties of the adhesive or sealant composition itself, but also on the conditions of application of the adhesive or sealant layer. In particular, it has been observed that adhesive/sealing performance greatly varies depending on the quality of the contact between the adhesive or sealant layer and the underlying tissue, such quality of contact being influenced by the device used to deposit the adhesive or sealant layer.

Document US 2005/0127119 discloses for instance an applicator that may be connected to a dispensing appliance for applying adhesive or sealant to biological tissue, such as lung or liver tissue. The applicator has an adapter for connection to an outlet of a mixer or dispensing appliance, and a dispensing slot. The applicator has an enclosure extending from the adapter to the slot, such an enclosure having a curved shape so that the side of the enclosure which is turned towards the glued substrate forms an angle with the longitudinal axis of the adapter. In addition, the enclosure is conically enlarged towards the dispensing slot. According to this document, due to the curvature of the enclosure, the tissue that is to be glued, e.g. lung tissue, is first smoothened and immediately thereafter evenly coated with adhesive or sealant.

Document US 6 063 055 A discloses another applicator for applying adhesive or sealant to biological tissue.

However, in practice, it has proven to be difficult to obtain reproducible adhesive/sealing performance, even when using such an applicator for applying adhesive or sealant on large vessel.

### SUMMARY OF THE INVENTION

One aim of the invention is to provide an applicator for depositing a layer of adhesive or sealant composition on a biological and/or prosthetic tissue, which allows to obtain an improved adhesion between the layer of adhesive or sealant and the biological and/or prosthetic tissue.

This aim is achieved according to the present invention by an applicator for depositing a layer of adhesive or sealant composition on a target site of a biological and/or prosthetic tissue, the applicator comprising a body having an application face intended to be positioned facing the biological and/or prosthetic tissue, and comprising:
- a first inlet opening for allowing the adhesive or sealant composition to flow from a dispensing device into the applicator,
- a first outlet opening provided in the application face, the first outlet opening being shaped as a slot for allowing the adhesive or sealant composition to flow out of the applicator and to form the layer of adhesive or sealant composition,
- a first inner channel for guiding the adhesive or sealant composition from the first inlet opening to the first outlet opening,
- a second inlet opening provided in the application face, adjacent the first outlet opening, for allowing pollutants or undesirable elements to be removed from the target site while depositing the layer of adhesive or sealant composition,
- a second outlet opening for allowing the removed pollutants or undesirable elements to be evacuated from the applicator,
- a second inner channel for guiding the removed pollutants or undesirable elements from the second inlet opening to the second outlet opening,

wherein the first outlet opening has a first section and the second inlet opening has a second section, the second section being greater than the first section,
wherein the first outlet opening shaped as a slot has a first width and a first height, and the second inlet opening has a second width and a second height, wherein the second width is equal or greater than the first width.

With such an applicator, elimination of pollutants or undesirable elements (e.g. fluid, material or tissue fragments) from the target site and deposition of the adhesive or sealant composition can be made simultaneously.

More precisely, while the surgeon displaces the applicator along the target site, the target site is progressively cleaned and the layer of adhesive or sealant composition is deposited immedialty after cleaning. This promote adhesion of the deposited layer with the surface of the target site.

In addition, during deposition, a depression is created between the surface of the target site and the adhesive or sealant composition. As a result, an intimate contact between the surface of the target site and the layer of adhesive or sealant composition may be obtained despite irregularities on the surface of the tissue.

According to particular embodiments of the invention, the applicator may also have at least one of the following features:
- the first outlet opening shaped as a slot, has a first width which is comprised between 2 and 15 millimeters, preferably comprised between 5 and 8 millimeters,
- the first outlet opening shaped as a slot, has a first height which is comprised between 0.2 and 1 millimeters, preferably between 0.4 and 0.6 millimeters,
- the second inlet opening has a second height which is comprised between 0.2 and 5 millimeters, preferably between 0.3 and 0.5 millimeters,
- the first inlet opening has a circular shape and the first inner channel has a cross section having a width which continuously increases from the first inlet opening to the first outlet opening and having a height which continuously decreases from the first inlet opening to the first outlet opening,
- the body has a first attachment portion adapted for cooperating with an attachment portion of the dispensing device in order to attach the applicator to the dispensing device, by translating and/or rotating the first attachment portion of the body relative to the attachment portion of the dispensing device according to a main axis of attachment,
- the first attachment portion comprises a male conical outer surface adapted to fit with a female conical inner surface of the attachment portion of the dispensing device according to the main axis of attachment, and lugs protruding at right angle relative to the main axis of attachment for locking the first attachment portion of the body to the attachment portion of the dispensing device,
- the application face is planar and forms an angle with the main axis of attachment, the angle being comprised between 90 and 30 degrees, preferably between 90 and 60 degrees, more preferably between 75 and 85 degrees,
- the body comprises a wall separating the first inner channel and the second inner channel, the wall having a thickness, measured between the first outlet opening and the second inlet opening which is comprised between 25 micrometers and 1 centimeter,
- the first inner channel has a first main axis, and the second inner channel has a second main axis, the second main axis being oriented relative to the first main axis with an angle comprised between 10 and 20 degrees,
- the body is formed in one single piece of material,
- the material is transparent or translucent to visible light so that an observer can see the adhesive or sealant composition flowing within the first inner channel,
- the material is opaque to radiations having a wavelength comprised between 350 and 670 nanomaters, preferably of 405 nanometers.

The invention also relates to an applicator assembly comprising:
- a dispensing device comprising a reservoir filled with an adhesive or sealant composition, and having an end opening for allowing the adhesive or sealant composition to flow out of the dispensing device, and
- an applicator as defined previously, adapted to be attached to the dispensing device in order to allow depositing a layer of adhesive or sealant composition flowing from the end opening of the dispensing device on a biological and/or prosthetic tissue.

According to an embodiment of the invention, the applicator assembly comprises a suction tube and the applicator comprises a second attachment portion for attaching an end of the suction tube to the applicator in order to connect the second inner channel to a suction apparatus.

According to an embodiment of the invention, the adhesive or sealant composition contained in the reservoir comprises a pre-polymer comprising a polymeric unit of the general formula (-A-B-)ₙ, wherein A represents a substituted or un-substituted ester, B represents a substituted or unsubstituted acid ester comprising at least two acid ester functionalities, and n represents an integer greater than 1.

Component A may be derived from a polyol, such as a diol, triol, tetraol or greater. Suitable polyols include diols, such as alkane diols; triols, such as glycerol, trimethylolpropane, triethanolamine; tetraols, such as erythritol, pentaerythritol; and higher polyols, such as sorbitol. Unsaturated diols, such as tetradeca-2,12-diene-1,14-diol, or other diols including macromonomer diols such as, for example polyethylene oxide, and N-methyldiethanoamine (MDEA) can also be used. Preferably, the polyol is substituted or unsubstituted glycerol.

Component B may be derived from a polyacid, such as a diacid or higher order acid. A wide variety of diacid, or higher order acids, can be used. Exemplary acids include, but are not limited to, citric acid (3 carbons), glutaric acid (5 carbons), adipic acid (6 carbons), pimelic acid (7 carbons), sebacic acid (8 carbons), and azelaic acid (nine carbons). Exemplary long chain diacids include diacids having more than 10, more than 15, more than 20, and more than 25 carbon atoms. Non-aliphatic diacids can also be used. For example, versions of the above diacids having one or more double bonds can be used to produce polyol-diacid co-polymers. Preferably the diacid is substituted or unsubstituted sebacic acid.

Several substituents, such as amines, aldehydes, hydrazides, acrylates and aromatic groups, can be incorporated into the carbon chain. Exemplary aromatic diacids include terephthalic acid and carboxyphenoxypropane. The polyacids, e.g. the diacids, can also include substituents as well. For example, reactive groups like amine and hydroxyl can be used to increase the number of sites available for cross-linking. Amino acids and other biomolecules can be used to modify the biological properties. Aromatic groups, aliphatic groups, and halogen atoms can be used to modify the interchain interactions within the polymer.

The pre-polymer may further comprise a polyamide or polyurethane backbone. For example, polyamine (comprising two or more amino groups) may be used to react with polyacid together with polyol or after reacting with polyol. In other examples, polyisocianates (comprising two or more isocyanate groups) may be used to react with polyacid together with polyol or after reacting with polyol.

The pre-polymer is preferably able to be activated. It can be activated by introducing functional groups that can react or be reacted to form crosslinks. Suitable functional groups to be activated on the pre-polymer backbone include hydroxy groups, carboxylic acid groups, amines, and combinations thereof, preferably hydroxy and/or carboxylic acid. The free hydroxyl or carboxylic acid groups on the pre-polymer can be activated by functionalizing the hydroxy groups with a moiety which can form a crosslink between polymer chains. The groups that are activated can be free hydroxyl or carboxylic acid groups on A and/or B moieties in the pre-polymer. Preferably, the functional group is or contains an acrylate group.

Acrylate groups are moieties containing substituted or unsubstituted acryloyl group. The acrylate may contain the following group: -C(=O)-CR1=CR2R3, wherein R1, R2, R3 are independently from one another, selected in the group consisting of H, alkyl such as methyl or ethyl, aryl such as phenyl, substituted alkyl, substituted aryl, carboxylic acid, ester, amide, amine, urethane, ether, and carbonyl. Preferably, R1, R2 and R3 are H; or R1 is CH3, R2 and R3 are H; or R1 and R2 are H and R3 is CH3; or R1 and R2 are H and R3 is phenyl.

According to a preferred embodiment, the adhesive or sealant composition contained in the reservoir comprises a crosslinkable polyester pre-polymer comprising a polymeric unit of the general formula (-A-B-)n, wherein A represents a substituted or un-substituted ester, B represents a substituted or un-substituted acid ester comprising at least two acid ester functionalities, and n represents an integer greater than 1 and wherein A comprises an acrylate group prior to crosslinking.

According to a preferred embodiment, the adhesive or sealant composition comprises a pre-polymer comprising a polymeric unit of the general formula (-A-B-)n, wherein (i) the content of grafted anhydride in the composition is less than 0.05 mol/mol of polyacid, or (ii) the composition comprises an anhydride compound.

The activated pre-polymer may have the general formula: 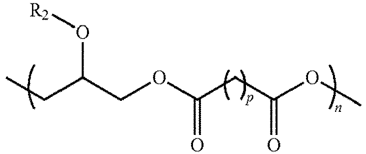 wherein n and p each independently represent an integer equal or greater than 1, and wherein R2 in each individual unit represents hydrogen or a polymer chain or -C(=O)-CR3=CR4R5, wherein R3, R4, R-5 are independently from one another, selected in the group consisting of H, alkyl such as methyl or ethyl, aryl such as phenyl, substituted alkyl, substituted aryl, carboxylic acid, ester, amide, amine, urethane, ether, and carbonyl. Preferably, R3, R4 and R5 are H; or R3 is CH3, R4 and R5 are H; or R3 and R4 are H and R5 is CH3; or R3 and R4 are H and R5 is phenyl. Preferably, p is an integer from 1-20, more preferably from 2-10, even more preferably from 4-10. It is most preferred when p=8.

In particular, the pre-polymer may have the following structure: 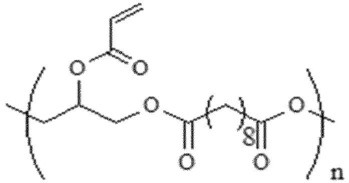 wherein n represents an integer equal or greater than 1.

According to a preferred embodiment, the adhesive or sealant composition is a light-curable compound. "Light curable compound" refers to compounds that are configured to polymerize or otherwise cure upon receiving appropriate radiant energy, more particularly in the form of light from a light source.

Preferably, the light-curable compound comprises a pre-polymer and a photoinitiator, said photoinitiator being able to induce polymerization of the said pre-polymer when exposed to light of a specific wavelength.

According to a special embodiment, said photoinitiator is sensitive to ultraviolet (UV) radiations.

Examples of suitable photoinitiators sensitive to UV radiations include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof.

According to another embodiment, said photoinitiator is sensitive to visible light (typically blue light or green light).

Examples of photoinitiators sensitive to visible light include, but are not limited to: diphenyl(2,4,6-trimethylbenzoyl)-phosphine oxide, eosin Y disodium salt, N-Vinyl-2-Pyrrolidone (NVP) and triethanolamine, and camphorquinone.

In addition, the adhesive or sealant composition may also contain one or more pharmaceutical, therapeutic, prophylactic, and/or diagnostic agents that can be released during the time period that the material functions as a sealant/adhesive. The agent may be a small molecule agent, for example having molecular weight less than 2000, 1500, 1000, 750, or 500 Da, a biomolecule, for example peptide, protein, enzyme, nucleic acid, polysaccharide, growth factors, cell adhesion sequences such as RGD sequences or integrins, extracellular matrix components, or combinations thereof. Similarly, the adhesive or sealant composition may also contain cells or biological tissue or particulate bone grafts or/and bone substitutes. Exemplary classes of small molecule agents include, but are not limited to, anti-inflammatories, analgesics, antimicrobial agents, and combinations thereof. Exemplary growth factors include, without limitation, TGF-□, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids. Exemplary extracellular matrix components include, but are not limited to, collagen, fibronectin, laminin, elastin and combinations thereof.

According to an embodiment of the invention, the dispensing device comprises a syringe having a cylinder defining the reservoir, and a piston slidably mounted within the cylinder, and comprising a casing adapted to be arranged around the cylinder so as to prevent light from irradiating the adhesive or sealant composition contained within the reservoir.

According to an embodiment of the invention, the casing comprises resilient tabs which are adapted to be deformed upon insertion of the cylinder within the casing and to abut against a shoulder of the syringe once the cylinder has been inserted in the casing, so as to lock the casing on the cylinder.

According to an embodiment of the invention, the applicator assembly comprises a packaging for enclosing the applicator and the dispensing device, the packaging being made of a material preventing penetration of bacteria.

The disclosure also relates to a method not forming part of the claimed invention for holding two parts of biological and/or prosthetic tissues together, comprising steps of:
- joining the two parts together, possibly by sewing with a surgical suture or with staples or edge-to-edge,
- applying a layer of adhesive or sealant composition over the junction by using an applicator assembly as defined previously, and
- exposing the layer of adhesive or sealant composition to light so as to induce cross-linking of the adhesive or sealant composition.

According to an embodiment, the two parts which are joined together comprise a blood vessel and a vascular prosthesis, preferably made of a synthetic material such as a Dacron or Teflon^{®}, or of a biological material.

According to an embodiment, the two parts comprise a blood vessel and a tubular vascular prosthesis, and are joined together by termino-terminal anastomosis or by termino-lateral anastomosis.

The disclosure further relates to a method not forming part of the claimed invention for adhering or sealing targeted surfaces including biological and/or prosthetic tissue comprising applying a layer of adhesive or sealant composition to the targeted surfaces by using an applicator assembly as defined previously.

According to special embodiment, said prosthetic tissue is graft material such as PTFE-based graft /patch material, Dacron-based graft / patch material or any combination thereof.

The disclosure further relates to a method not forming part of the claimed invention for making a tissue adhere to a medical device, the method comprising applying a layer of adhesive composition to a surface of the tissue and/or of the medical device by using an applicator assembly as defined previously.

The disclosure further relates to a method not forming part of the claimed invention for making a medical device adhere to another medical device, or for making parts of medical devices adhere together, by using an applicator assembly as defined previously.

The disclosure also relates to a method not forming part of the present invention for forming a barrier or for filling a void, especially in order to prevent leaking, e.g. of blood or any other fluids, gas, etc, said method comprising applying a layer of adhesive or sealant composition to a surface by using an applicator assembly as defined previously.

Examples of use include stopping bleeding, for example, due to a wound or trauma or during surgery such as after suturing a graft to a vessel or after vascular access in endovascular procedures.

Other examples include using a layer of adhesive or sealant composition as adjunct to sutures in vascular anastomosis, and as adjunct to sutures in ePTFE grafts, e.g. ePTFE vascular grafts.

Other examples include using a layer of adhesive or sealant composition as passive barrier for example in case of :
- cell regeneration and tissue repair : tissue ingrowth prevention so that only the desirable cells repopulate the protected space thus promoting tissue repair; mechanical protection and stabilization of cell regenerating region, or
- tissue to tissue adhesion prevention, following surgical procedures especially at the thoracic and abdominal levels.

The method comprising applying a layer of adhesive or sealant composition to a surface by using an applicator assembly as defined previously can be applied in a variety of other situations where an adhesive or sealant is required. These include, but are not limited to, air leaks following a lung resection; to seal gastrointestinal leaks; to reduce the time for surgical procedures; to seal dura; to ease laparoscopic procedures; as a degradable skin adhesive; as a hernia matrix to prevent or to reduce the need for stables or tacks; to prevent blood loss; to manipulate organs or tissues during surgical procedures; to secure corneal transplants in place; to patch a heart to deliver drugs and/or to reduce dilation of the heart after myocardial infarction; to attach another material to a tissue; to augment sutures or staples; to distribute forces across tissue; to prevent leaks; as a barrier membrane on the skin to prevent evaporation of water from burnt skin; as a patch for delivery of antiscar or antimicrobial medication; to attached devices to tissue; to attach devices to mucus membrane as a tape to secure devices within an oral cavity, such as to hold dentures and oral appliances; as a tape to anchor soft tissue to bone; as moldable barrier in guided tissue (e.g. bone repair and/or regeneration) and, preventing the formation of holes in tissue, enhancing/augmenting mechanical properties of tissues, etc.

The disclosure also relates to a method not forming part of the present invention for holding two parts of biological and/or prosthetic tissues together, comprising steps of:
- joining the two parts together, for instance by sewing with a surgical suture or with staples or edge-to-edge,
- removing blood or any other fluids from the target site and applying a layer of adhesive or sealant composition over the junction by using an applicator assembly as defined previously.

The thickness of the layer of adhesive or sealant composition can be at least about 50 microns, 100 microns, 500 microns or 1000 microns. Preferably it is below 1500 microns.

According to preferred embodiments, one of the above-mentioned methods may comprise steps of:
- joining the two parts together, for instance by sewing with a surgical suture or with staples or edge-to-edge,
- removing blood or any other fluids from the target site and applying a layer of adhesive or sealant composition over the junction by using an applicator assembly as defined above.

According to preferred embodiments, one of the above mentioned methods is applied to guided bone regeneration (GBR). GBR is a surgical procedure that uses barrier membranes with or without particulate bone grafts or/and bone substitutes. Osseous regeneration by guided bone regeneration depends on the migration of pluripotent and osteogenic cells (e.g. osteoblasts derived from the periosteum and/or adjacent bone and/or bone marrow) to the bone defect site and exclusion of cells impeding bone formation (e.g. epithelial cells and fibroblasts). The method may comprise steps of :
- applying bone graft material (e.g. autogenous bone, allografts, xenografts or alloplasts) to the target site,
- removing blood or any other fluids from the target site and applying a layer of adhesive or sealant composition over the bone graft material by using an applicator assembly as defined above.

According to preferred embodiments, one of the above mentioned methods may further comprise a step of:
- exposing the applied layer of adhesive or sealant composition to radiations so as to induce polymerization or curing of the adhesive or sealant composition.

According to some embodiments, the adhesive or sealant composition is irradiated with ultraviolet (UV) light in the presence of a photoinitiator to facilitate polymerization or curing.

Examples of suitable photoinitiators include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof.

In other embodiments, the adhesive or sealant composition is irradiated with visible light (typically blue light or green light) in the presence of a photoinitiator to facilitate polymerization or curing.

Examples of photoinitiators sensitive to visible light include, but are not limited to: diphenyl(2,4,6-trimethylbenzoyl)-phosphine oxide, eosin Y disodium salt, N-Vinyl-2-Pyrrolidone (NVP) and triethanolamine, and camphorquinone.

Irradiation is applied with a light source that is configured to transmit, emit, direct, or otherwise apply, light to a desired application site within the patient.

According to special embodiments, the light source is arranged in a separate light applicator.

The term "light source" refers to any light generating device, including, but not limited to, a halogen bulb, light-emitting diode (LED), LED array, and combinations thereof.

The two parts which are joined together may comprise :
- tissue edges, or
- a blood vessel and a vascular prosthesis, preferably made of a synthetic material such as a Dacron or ePTFE (GoreTex^{®}), or
- a blood vessel and a vascular prosthesis made of a biological material.
- two blood vessel parts, including artery and/or vein,
- a blood vessel and a prosthetic tissue, such as a patch or a stent.

The two parts may comprise a blood vessel and a tubular vascular prosthesis, and may be joined together by termino-terminal anastomosis or by termino-lateral anastomosis.

According to other embodiments, the layer of adhesive or sealant composition may be applied over the junction with no suture, e.g. for sutureless closure of incisions, e.g. vascular incisions.

Vascular incisions include for example vascular incisions of from about 0.5 to 6 millimeters, typically from about 4 to 5 millimeters.

Other types of wounds that can be treated include, but are not limited to, wounds that leak, wounds that are hard to close or that fail to heal properly through normal physiologic mechanisms.

The above-mentioned methods may be performed both inside and outside the body, for human or veterinary use. These methods may be performed under open surgery, minimally invasive surgery or laparoscopic surgery.

### BRIEF DESCRIPTION OF THE DRAWINGS

Embodiments ot the invention will be described with reference to the drawings, in which:
- figures 1 and 2 schematically show an applicator according to a first embodiment of the invention,
- figure 3 is a cross section view of the applicator of figures 1 and 2,
- figures 4 and 5 schematically show an applicator according to a second embodiment of the invention,
- figure 6 schematically shows an applicator according to an example not forming part of the claimed invention,
- figure 7 schematically shows an applicator according to a further example not forming part of the claimed invention,
- figures 8 and 9 schematically show an applicator according to a fifth embodiment of the invention,
- figure 10 is a cross section view of the applicator of figures 8 and 9,
- figure 11 schematically shows a dispensing device to which the applicator may be attached,
- figure 12 schematically shows a dispensing device with a casing,
- figure 13 schematically shows a packaging for enclosing the applicator and the dispensing device,
- figure 14 schematically shows an applicator assembly according to one embodiment of the invention.

### DETAILLED DESCRIPTION OF PREFERRED EMBODIMENTS

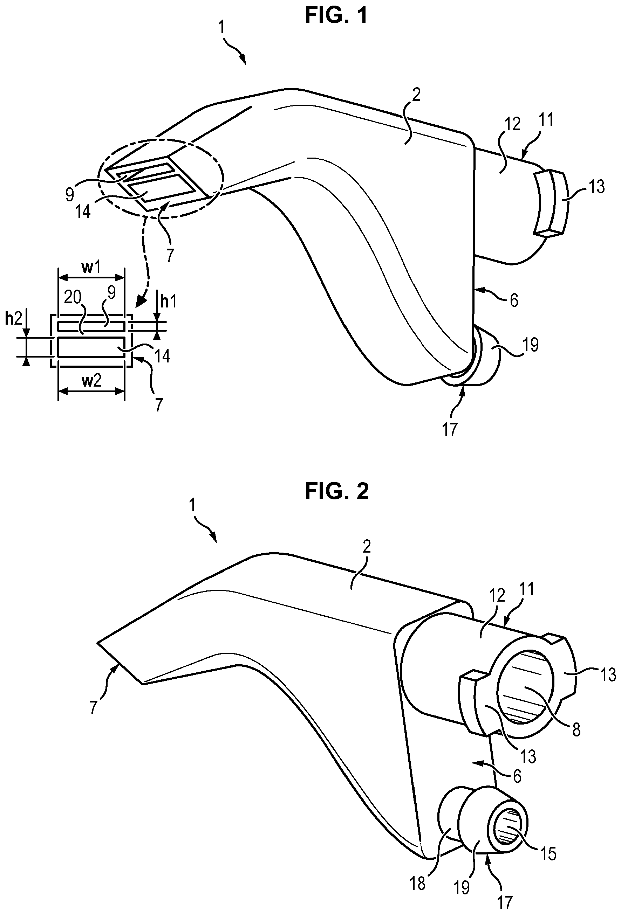

Refering to figures 1 to 3, according to a first embodiment, the applicator 1 is made in one single piece of material. The material of the applicator 1 may be a biocompatible material, whether rigid or flexible. The material of the applicator 1 can be for instance a polycarbonate which is a rigid material compatible with injection molding manufacturing technique.

The material of the applicator 1 is translucent to visible light so that an observer can see the adhesive or sealant composition flowing through the applicator 1. However, the material of the applicator 1 is preferably opaque to radiations at wavelengths that activate the adhesive or sealant composition.

The applicator 1 comprises a body 2 comprising an attachment portion 3, a delivery portion 4 and an intermediate portion 5 connecting the attachment portion 3 to the delivery portion 4.

The attachment portion 3 has an attachment face 6 which is intended to be connected to a dispensing device.

The delivery portion 4 has an application face 7 which is intended to be positioned facing the biological and/or prosthetic tissue where the adhesive or sealant composition is to be deposited. The application face 7 is substantially planar.

The intermediate portion 5 is bent.

The body 2 further comprises a first inlet opening 8 provided on the attachment face 6, a first outlet opening 9 provided on the application face 7 and a first inner channel 10 extending from the first inlet opening 8 to the first outlet opening 9.

The first inlet opening 8 has a circular shape. The diameter of the first inlet opening 8 may be comprised between 4 millimeters and 4.5 millimeters, preferably between 4.270 millimeters and 4.315 millimeters, for instance 4.29 millimeters. The first inlet opening 8 is adapted to allow adhesive or sealant composition to flow from the dispensing device into the applicator 1.

The first outlet opening 9 is shaped has a slot for allowing the adhesive or sealant composition to flow out of the applicator 1 and to form the layer of adhesive or sealant composition. The first outlet opening 9 has a first width w₁ and a first height h₁. The first width w₁ is comprised between 2 and 15 millimeters, preferably comprised between 5 and 8 millimeters, for instance equal to 3.89 millimeters. The first height h₁ is comprised comprised between 0.2 and 1 millimeters, preferably between 0.4 and 0.6 millimeters, for instance 0.63 millimeters.

The first inner channel 10 is adapted for guiding the adhesive or sealant composition from the first inlet opening 8 to the first outlet opening 9. The first inner channel 10 has a cross section having a width w which continuously increases from the first inlet opening 8 to the first outlet opening 9 and having a height h which continuously decreases from the first inlet opening 8 to the first outlet opening 9.

The body 2 also comprises a first attachment component 11 adapted for cooperating with an attachment component of a dispensing device in order to attach the applicator 1 to the dispensing device. The first attachment component 11 comprises a first annular wall 12 protruding from the attachment face 6 and surrounding the first inlet opening 8. The annular wall 12 comprises a male conical outer surface adapted to fit with a female conical inner surface of the attachment portion of the dispensing device according to a main axis of attachment X₁. The first attachment component 11 also comprises lugs 13 protruding at right angle relative to the main axis of attachment X₁ for locking the first attachment component 11 of the body 2 to the attachment component of the dispensing device.

The body 2 also comprises a second inlet opening 14 provided on the application face 7, a second outlet opening 15 provided on the attachment face 6 and a second inner channel 16 extending from the second inlet opening 14 to the second outlet opening 15.

The second inlet opening 14 has a rectangular shape. The second inlet opening 14 is located adjacent the first outlet opening 9 on the application face 7, for allowing pollutants or undesirable elements (e.g. fluid, material or tissue fragments) to be removed from the target site while the layer of adhesive or sealant composition is deposited through the first outlet opening 9. The second inlet opening 14 has a second width w₂ and a second height h₂. The second width w₂ is equal or greater than the first width w₁. The second width w₂ is comprised between 2 and 15 millimeters, preferably comprised between 5 and 8 millimeters, for instance equal to 4 millimeters. The second height h₂ is comprised between 0.2 and 5 millimeters, preferably between 0.3 and 2 millimeters, for instance 1.55 millimeters.

The second outlet opening 15 has a circular shape. The second outlet opening 15 has a diameter which is comprised between 1.6 millimeters and 10 millimeters for instance 2 millimeters. The second outlet opening 15 is adapted for allowing the pollutants or undesirable elements (e.g. fluid, material or tissue fragments) removed from the target site to be evacuated towards an external suction apparatus.

The second inner channel 16 extends within the body, in parallel to the first inner channel 10.

The body 2 also comprises a second attachment component 17 adapted for attaching an end of a suction tube to the applicator 1 in order to connect the second inner channel 16 to the external suction apparatus. The second attachment component 17 comprises a second annular wall 18 protruding from the attachment face 6 and surrounding the second outlet opening 15. The second attachment component 17 also comprises a conical bulge 19 extending around the second annular wall 18. The conical bulge 19 is adapted to be inserted in an end opening of the suction tube in order to attach the suction tube to the applicator 1.

The body 2 comprises a wall 20 separating the first inner channel 10 from the second inner channel 16. The wall 20 has a thickness which decreases from the attachment face 6 to the application face 7. The wall 20 has a thickness measured between the first outlet opening 9 and the second inlet opening 14 (within the plane of the application face 7) which is comprised between 25 micrometers and 1 centimeter, for instance 0.46 millimeters.

The application face 7 forms an angle α with the main axis of attachment, the angle being comprised between 90 and 30 degrees, preferably between 90 and 60 degrees, more preferably between 75 and 85 degrees, for instance 63.72 degrees.

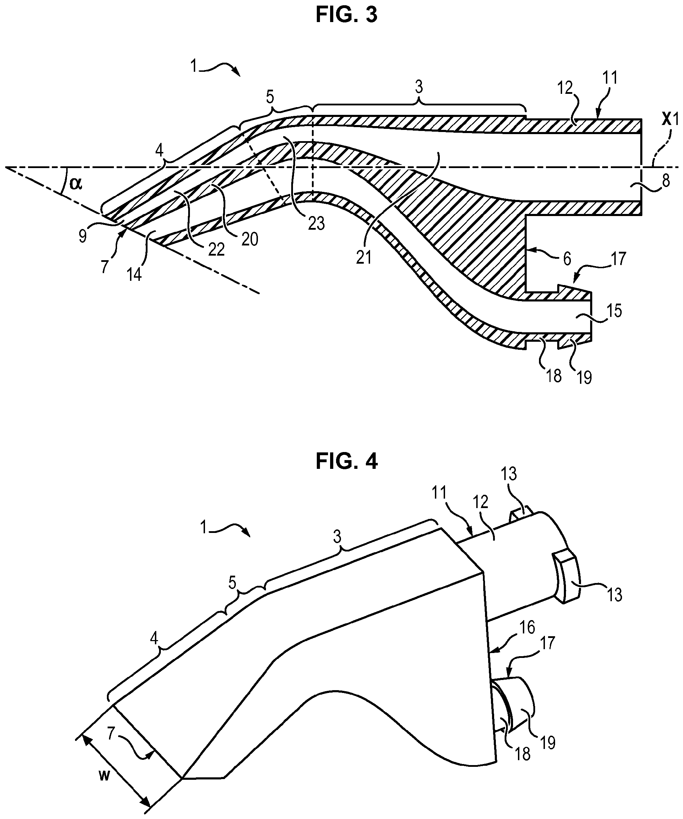

As can been seen from figure 3, the first channel 10 has an inlet section 21, an outlet section 22 and an intermediate section 23 connecting the inlet section 21 to the outlet section 22. The inlet section 21 entends within the attachment portion 3 of the body 2. The inlet section 21 extends from the first inlet opening 8 to the intermediate section 23. The outlet section 22 entends within the delivery portion 4 of the body 2. The outlet section 22 extends from the intermediate section 23 to the first outlet opening 9. The intermediate section 23 extends within the intermediate portion 5 of the body 2. The intermediate section 23 is curved while the oulet section 22 is substantially straight.

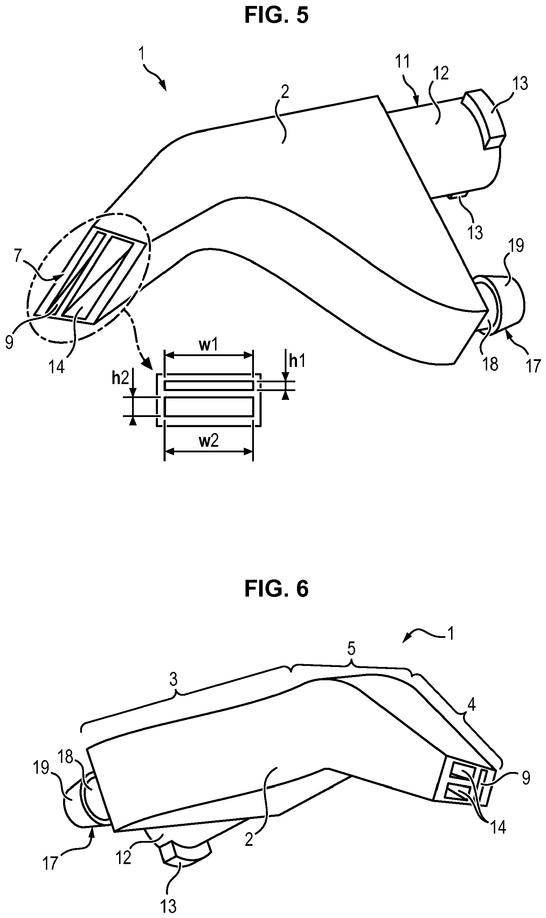

Figures 4 and 5 schematically show an applicator 1 according to a second embodiment of the invention.

In this second embodiment, the applicator 1 is similar to the applicator of the first embodiment, except that the delivery portion 4 of the applicator 1 has a width W which continuously increases from the intermediate portion 5 towards the application face 7.

In this second embodiment, the first outlet opening 9 has a first width w₁ which is equal to 9.29 millimeters and a first height h₁ which is equal to 0.52 millimeters

The second inlet opening 14 has a second width w₂ and a second height h₂. The second width w₂ is greater than the first width w₁. The second width w₂ is equal to 8.87 millimeters and the second height h₂ is equal to 1.54 millimeters.

This second embodiment allows depositing a layer of adhesive or sealant composition which has a larger width than the layer of adhesive or sealant composition deposited with the first embodiment. This second embodiment may be adapted for depositing a layer of adhesive or sealant composition on large blood vessels, such as aorta, femoral, carotid or coronary arteries.

Figure 6 schematically shows an applicator 1 according to an example not forming part of the claimed invention.

In this example, the delivery portion of the applicator has a width which continuously decreases from the attachment portion towards the application face.

In this example, the first outlet opening has a first width which is equal to 2.38 millimeters and a first height which is equal to 0.40 millimeters.

In addition, the applicator 1 comprises two second inlet openings 14 arranged side by side, adjacent the first outlet opening 9 on the application face 7. This allows for filtering material of a predetermined size, and prevent undesirable removal of material such as biomaterial or cells (for example bone powder or cells).

In this example, the body 2 has an intermediate portion 5 which is bent. The intermediate portion 5 has a radius of curvature which extends within a main plane of the slot 9.

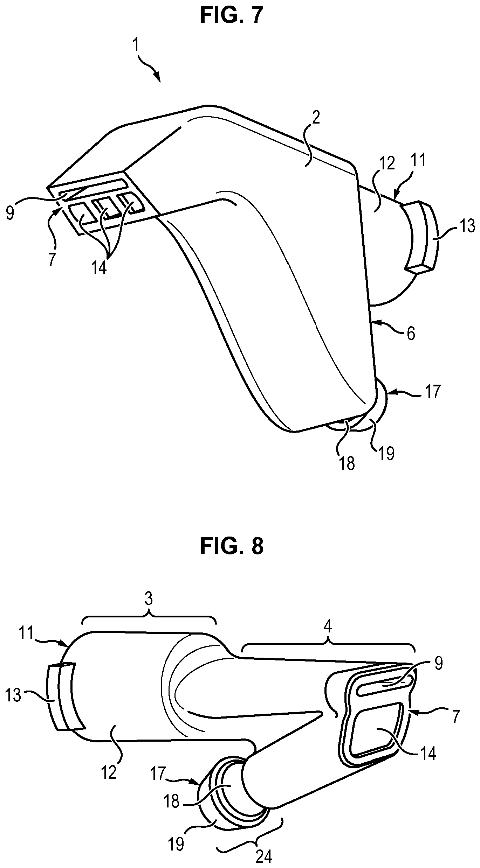

Figure 7 schematically shows an applicator 1 according to a further example not forming part of the claimed invention.

In this further example, the applicator 1 has three second inlet openings 14 arranged side by side, adjacent the first outlet opening 9 on the application face 7.

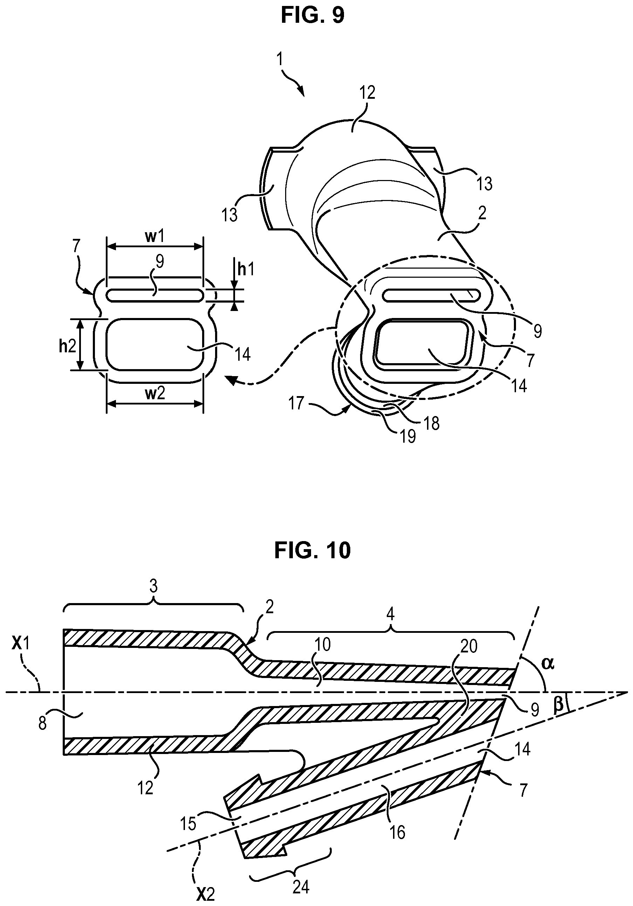

Refering to figures 8 to 10, according to a fifth embodiment, the applicator 1 comprises a body 2 comprising a first attachement portion 3, a second attachement portion 24 and a delivery portion 4.

The delivery portion 4 has an application face 7 which is intended to be positioned facing the biological and/or prosthetic tissue where the adhesive or sealant composition is to be deposited. The application face 7 is substantially planar.

The body 2 comprises a first inlet opening 8 provided on the first attachment portion 3, a first outlet opening 9 provided on the delivery portion 4 and a first inner channel 10 extending from the first inlet opening 8 to the first outlet opening 9.

The first outlet opening 9 is provided on the the application face 7.

The first inlet opening 8 has a circular shape. The diameter of the first inlet opening 8 is comprised between 4 millimeters and 4.5 millimeters, preferably between 4.270 millimeters and 4.315 millimeters, for instance 4.28 millimeters. The first inlet opening 8 is adapted to allow adhesive or sealant composition to flow from the dispensing device into the applicator 1.

The first outlet opening 9 is shaped has a slot for allowing the adhesive or sealant composition to flow out of the applicator and to form the layer of adhesive or sealant composition. The first outlet opening has a first width w₁ and a first height h₁. The first width w₁ is comprised between 2 and 15 millimeters, preferably comprised between 6 and 8 millimeters, for instance equal to 4 millimeters. The first height h₁ is comprised comprised between 0.2 and 1 millimeters, preferably between 0.4 and 0.6 millimeters, for instance 0.5 millimeters .

The first inner channel 10 is adapted for guiding the adhesive or sealant composition from the first inlet opening 8 to the first outlet opening 9. The first inner channel 10 has a cross section having a width w which continuously increases from the first inlet opening 8 to the first outlet opening 9 and has a height h which continuously decreases from the first inlet opening 8 to the first outlet opening 9.

The first attachment portion 3 is adapted for cooperating with an attachment portion of a dispensing device in order to attach the applicator 1 to the dispensing device. The first attachment portion 3 comprises a first annular wall 12 surrounding the first inlet opening 8. The annular wall 12 comprises a male conical outer surface adapted to fit with a female conical inner surface of the attachment portion of the dispensing device according to a main axis of attachment X₁. The first attachment portion 3 also comprises lugs 13 protruding is radial directions relative to the main axis of attachment X₁ for locking the first attachment portion 12 of the body 2 to the attachment portion of the dispensing device.

The body 2 also comprises a second inlet opening 14 provided on the delivery portion 4, a second outlet opening 15 provided on the second attachment portion 24, and a second inner channel 16 extending from the second inlet opening 14 to the second outlet opening 15.

The second inlet opening 14 is provided on the application face 7.

The second inlet opening 14 has a substantially rectangular shape. The second inlet opening 14 is located adjacent the first outlet opening 9 on the application face 7, for allowing pollutants or undesirable elements (e.g. fluid, material or tissue fragments) to be removed from the target site though the second inlet opening 14 while the layer of adhesive or sealant composition is deposited through the first outlet opening 9. The second inlet opening 14 has a second width w₂ and a second height h₂. The second width w₂ is equal or greater than the first width w₁. The second width w₂ is comprised between 2 and 15 millimeters, preferably comprised between 5 and 8 millimeters, for instance equal to 3.72 millimeters. The second height h₂ is comprised between 0.2 and 5 millimeters, preferably between 0.3 and 0.5 millimeters, for instance 2.32 millimeters.

The second outlet opening 15 has a circular shape. The second outlet opening 15 has a diameter which is comprised between 4 and 8 millimeters, for instance 5.05 millimeters. The second outlet opening 15 is adapted for allowing the pollutants or undesirable elements (e.g. fluid, material or tissue fragments) removed from the target site to be evacuated towards an external suction apparatus.

The second attachment portion 24 is adapted for attaching an end of a suction tube to the applicator 1 in order to connect the second inner channel 16 to the external suction apparatus. The second attachment portion 24 comprises a second annular wall 18 surrounding the second outlet opening 15. The second attachment portion 24 also comprises a conical bulge 19 extending around the second annular wall 18. The conical bulge 19 is adapted to be inserted in an end opening of the suction tube in order to attach the suction tube to the applicator 1.

The body 2 also comprises a wall 20 separating the first inner channel 10 and the second inner channel 16. The wall 20 has a thickness which decreases from the attachment portions 3, 24 to the application face 7. The wall 20 has a thickness measured between the first outlet opening 9 and the second inlet opening 14 (within the plane of the application face 7) which is comprised between 25 micrometers and 1 centimeter, for instance 1 millimeters.

The application face 7 forms an angle α with the main axis X₁ of attachment, the angle being comprised between 90 and 60 degrees, preferably between 75 and 85 degrees, for instance 70 degrees.

In this fifth embodiment, the first channel 10 is substantially straight and extend along a first main axis X₁. The second channel 16 is substantially straight and extends along a second main axis X₂. The second main axis X₂ is oriented relative to the first main axis X₁ with an angle β comprised between 10 and 20 degrees, for instance 18.72 degrees.

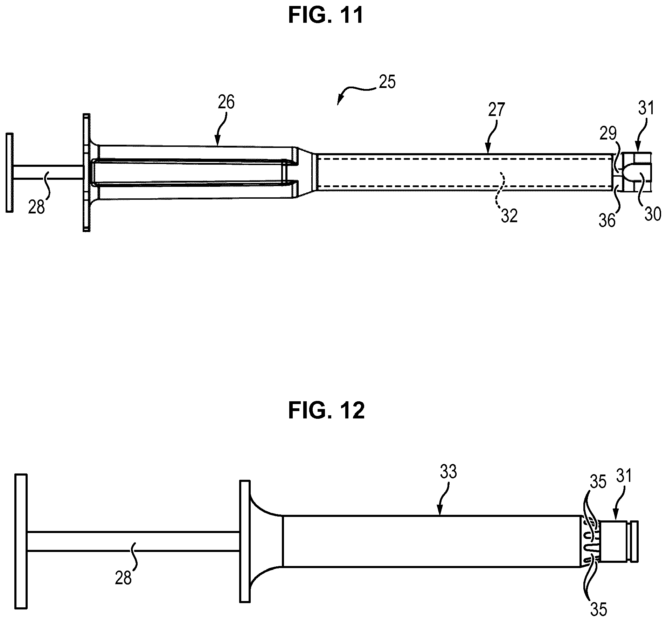

Figure 11 schematically shows a dispensing device 25 to which the applicator 1 may be attached,

The dispensing device 25 comprises a syringe 26. The syringe 26 comprises a cylinder 27 and a piston 28 arranged in the cylinder 27 so as to be able to slide within the cylinder 27 according to a longitudinal axis of the cylinder 27.

The syringe 26 also comprises a cannula 29 having an end opening 30 for allowing the adhesive or sealant composition to flow out of the dispensing device 25.

The syringe 26 comprises an attachment portion 31 surrounding the end opening 30. The attachment portion 31 of the syringe 26 is adapted to cooperate with the first attachment portion 3 of the applicator 1, in order to attach the applicator 1 to the dispensing device 25. More precisely, the attachment portion 31 of the dispensing device 25 has a female conical inner surface. The male conical outer surface of the first attachment portion 3 of the applicator 1 is adapted to fit with the female conical inner surface of the attachment portion 31 of the dispensing device 25.

The cylinder 27 and the piston 28 together define a reservoir 32 which may be filled with the adhesive or sealant composition.

The cylinder 27 is preferably made of a material which does not react with or does not diffuse within the adhesive or sealant composition. The material of the cylinder may be glass or polymer, such as cyclic olefin copolymer (COC) or cyclic olefin polymer (COP). The material of the cylinder may contain a substance, such as a colorant, which may filter light so as to prevent light at a predetermined wavelength from irradiating the adhesive or sealant composition contained within the reservoir and initiate cross-linking of the adhesive or sealant composition.

The adhesive or sealant composition is preferably a composition comprising a pre-polymer as disclosed in document WO 2014/190302, WO2016/202984 or WO2016/202985.

The adhesive or sealant composition may comprise a hydrophobic viscous pre-polymer having the formula A-B, wherein A is derived from a substituted or unsubstituted polyol moiety that may comprise one or acrylate groups prior to crosslinking, and B is derived from a substituted or unsubstituted diacid that may comprise acrylate groups prior to crosslinking. The adhesive or sealant composition has a viscosity which allows the prepolymer to stay in place at the site of administration without being washed away by bodily fluids, such as water and/or blood. According to preferred embodiment, the viscosity of the adhesive or sealant composition is comprised between 2 000 and 15000 centipoises at 37 degrees Celsius using a Brook Field Viscometer system.

The adhesive or sealant composition may also comprise a photoinitiator, which is able to initiate cross-linking of the pre-polymer when the adhesive or sealant composition is exposed to radiations at predetermined wavelength. Preferably, the adhesive or sealant composition comprises a prepolymer and a photoinitiator, said photoinitiator being able to induce polymerization of the said prepolymer when exposed at specific wavelength. According to special embodiment, said photoinitiator is sensitive to ultraviolet (UV) light. Examples of suitable photoinitiators sensitive to UV light include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof. According to another embodiment, said photoinitiator is sensitive to visible light (typically blue light or green light). Examples of photoinitiators sensitive to visible light include, but are not limited to: diphenyl(2,4,6-trimethylbenzoyl)-phosphine oxide, eosin Y disodium salt, N-Vinyl-2-Pyrrolidone (NVP) and triethanolamine, and camphorquinone.

Figure 12 schematically shows a dispensing device 25 provided with a casing 33.

The casing 33 is arranged around the cylinder 27 so as to prevent light from irradiating the adhesive or sealant composition contained within the reservoir 32.

The casing 33 comprises a tubular wall 34 surrounding the cylinder 27 and resilient tabs 35 extending from the tubular wall 34. The resilient tabs 35 are adapted to be deformed upon insertion of the cylinder 27 within the casing 33 and to abut against a shoulder 36 of the syringe 26 once the cylinder 27 has been inserted in the casing 33, so as to lock the casing 33 on the cylinder 27.

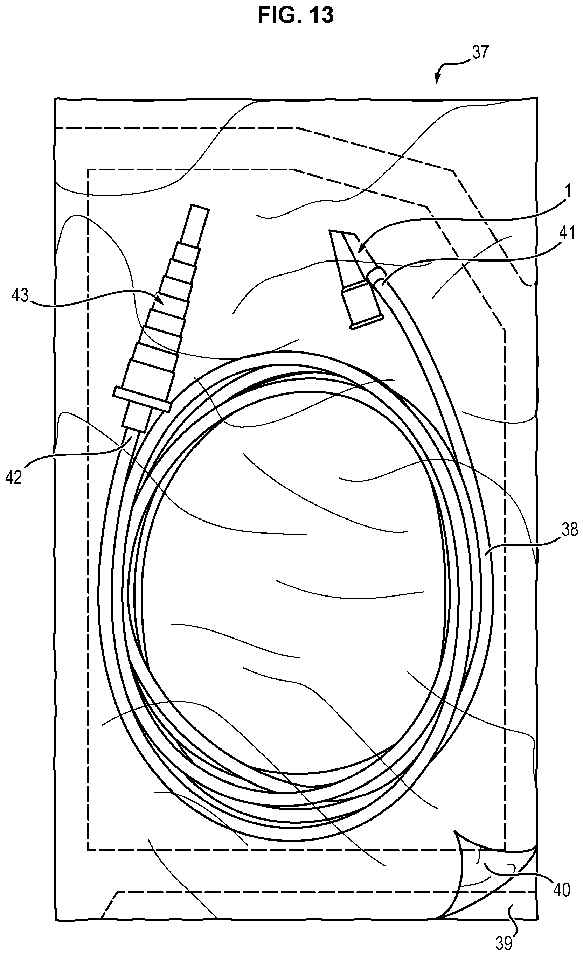

Figure 13 schematically shows a packaging 37 enclosing the applicator 1 and a suction tube 38 connected to the applicator 1.

The packaging 37 may also enclose the dispensing device 35 and optionally the casing 33.

The packaging 37 comprises two sheets 39 and 40 which are heatsealed together along their edge so as to define a pocket containing the applicator 1 and the suction tube 38. Each sheet 38, 40 is made of a material preventing penetration of bacteria. The first sheet 39 (or backing) may be made of a material which is permeable to gas, such as ethyle oxide (ETO), or may be a sheet of woven or unwoven synthetic fibers, for instance polyethylene fibers, such as Tyvek^{®} (provided by E. I. du Pont de Nemours and Company) so as to allow sterilization of the components contained in the packaging 37. The second sheet 40 may be made of a material which is impermeable to gas and which is transparent, such as polyethylene, polypropylene or a mixture therof.

The suction tube 38 is made of a material which is able to resist depression and which is biocompatible, such as polyurethane (PU) for instance.

As can be seen on figure 13, the suction tube 38 has a first end 41 and a second end 42. The applicator 1 is attached to the first end 41 of the suction tube 38.

The packaging 37 also contain a graduated (or stepped) connector 43 attached to the second end 42 of the suction tube 38 for connecting the suction tube 38 to a suction apparatus. The suction apparatus may be connected to a central medical vacuum supply by way of a pipeline system.

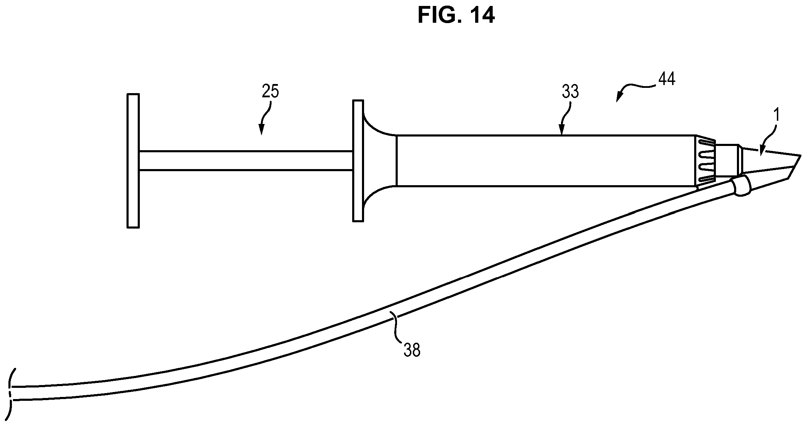

Figure 14 schematically shows an applicator assembly 44 according to one embodiment of the invention.

The applicator assembly 44 comprises a dispensing device 25, a casing 33 arranged around the dispensing device 25, an applicator 1 and a suction tube 38.

The applicator 1 is connected to the dispensing device 25.

More precisely, the first attachment portion 3 of the applicator 1 is connected to the attachment portion 31 of the dispensing device 25 in order to attach the applicator 1 to the dispensing device 25. Such a connection is made by translating and then rotating the first attachment portion 3 of the applicator 1 relative to the attachment portion 31 of the dispensing device according to a main axis of attachment X₁.

The applicator 1 is also connected to the suction tube 38.

More precisely, the second attachment portion 24 of the applicator 1 is connected to the first end 41 of the suction tube 38.

In use, the second end 42 of the suction tube 38 is connected to a suction apparatus via the stepped connector 43.

The applicator assembly 44 may be used to deposit a layer of adhesive or sealant so as to cover a tissue area, such as a junction between two parts. The first part may for instance comprise a blood vessel and the second part may comprise a vascular prosthesis.

The vascular prosthesis may a tubular vascular prosthesis. The tubular vascular prosthesis is made of a synthetic material such as a Dacron or ePTFE (e.g. GoreTex^{®}), or of a biological material.

The blood vessel and the tubular vascular prosthesis may have previously been joined together by termino-terminal anastomosis, for instance by sewing or by stapling, or are simply maintained edge-to-edge.

The applicator assembly 44 is positioned so that the first outlet opening 9 faces the blood vessel or the prosthesis, and extends transversally relative to the junction between the two parts.

Then, the piston 28 is caused to slide progressively within the cylinder 27 while the applicator 1 is simultaneously displaced along the junction line.

The adhesive or sealant composition flows into the first inner channel 10 of the applicator 1. At the same time, pollutants or undesirable elements are removed from the target site through the second inlet opening 14.

As a depression is created between the surface of the tissue and the adhesive or sealant composition, an intimate contact between the tissue and the layer of adhesive or sealant composition is obtained despite irregularities on the surface of the tissue.

As the material of the applicator 1 is translucent to visible light, the operator can see the adhesive or sealant composition flowing within the first inner channel 10 and detect the instant when the adhesive or sealant composition reaches the first outlet opening 9.

The adhesive or sealant composition flows out of the applicator 1 through the first outlet opening 9. This allows to deposit a layer of adhesive or sealant having the shape of a thin strip.

Once deposited, the adhesive or sealant layer is exposed to radiations so as to induce cross-linking of the pre-polymer.

The polymer of the cured adhesive or sealant layer is physically entangled with the underlying tissue. The adhesive or sealant layer thus provides a hemostatic seal and reinforces for example the junction between the blood vessel and the prosthesis.

The same method could be applied on a blood vessel and tubular vascular prosthesis which have been joined together by termino-lateral anastomosis.

Elimination of pollutants or undesirable elements (e.g. fluid, material or tissue fragments) from the target site facilitates deposition of the layer of adhesive or sealant composition. As a result, successful deposition of the adhesive or sealant composition may be obtained even in the case of complex junctions.

The same method could be also applied with a vascular prosthesis having the shape of a patch instead of a tubular prosthesis.

### EXAMPLE 1: Vascular surgery

An applicator assembly according to the invention has been used in vascular reconstruction procedure performed in porcine model. After clamping, a linear incision of 2-3 centimeters has been performed in the left carotid artery. An ePTFE (Gore-TEX^{®} Vascular Graft, standard-walled, 8 millimeters diameter) has been sutured over the incision. The artery has been declampled and blood leaks coming from the suture holes have been observed. The vessel was then re-clampled. A dispensing device including a 1 milliliter syringe containing PGSA (Poly(Glycerol) Sebacate Acrylate - US8143042) has been connected to an applicator according to figures 8 to 10. The suction tube was connected to an external suction apparatus connected to a vacuum line (the vacuum line is able to generate a depression of around - 0,8 bar). The PGSA was then applied over the suture line using the applicator. The applicator efficiently eliminated blood at the target site, enhancing the contact between the PGSA and the underlying tissue. After covering all the suture line, the PGSA was polymerized by applying radiations with Gecko's proprietary light activation pen (Setalum^{™}) for 30 seconds by square centimeter (s/cm²). When the vessel was declampled, hemostatis was obtained instantaneously.

### EXAMPLE 2: Urinary tract surgery

A 30 centimeters abdominal incision was performed in mini-pig model. Bladder and uretra were exposed. The bladder has been emptied before performing transversal incision to separate bladder and urethra, simulating radical proctectomy scenario. A Foley catheter (provided by BBraun) was placed through the uretra and deployed in the bladder. 4 sutures were applied in order to connect organs; blood and small urine quantities leaks from anastomosis region were observed. A dispensing device including 1 milliliter (mL) syringe containing PGSAA (Poly(Glycerol) Sebacate Acrylate Anhydride - WO2016202985) has been connected to an applicator according to the invention. The applicator was then connected to an external suction apparatus connected to a vacuum line (the vacuum line is able to generate a depression of around - 0,8 bar). The PGSAA has been applied over the anastomosis region. Polymerization of the PGSAA was performed by applying radiations with Gecko's proprietary light activation pen (Setalum^{™}) for 30 seconds per square centimeter (s/cm²). After polymerization, it was clearly possible to see the adhesive composition adhering to the target tissues and sutures. When pulling away the bladder from the uretra, one could clearly oberve that the PGSAA was restricting the separation of both tissues. After PGSAA application, no leak was observed coming from inside the anastomosis region.

The adhesive has then been removed and second application of adhesive prepolymer was performed again according to identical conditions but without vacuum.

This experiment has shown that use of vacuum :
a) Significantly improves usability of the applicator assembly by removing the liquids from the target tissue before applying the PGSAA; this results in enhanced deposition of the sealant/adhesive composition;
b) Significantly enhances the contact between the target surface and the sealant/adhesive therefore improving adhesion.

### EXAMPLE 3: Bone

This test was performed *ex vivo* using tranversal section of bovine femur. The periosteum of the bone was removed and 1,5 cm x 2,5 cm rectangles were cut for easier testing. The bone rectangles were hydrated by diving in Phosphate Buffer Saline. A dispensing device including a 1 milliliter (mL) syringe containing PGSA (Poly(glycerol) sebacate Acrylate) has then been connected to an applicator according to the invention. The applicator was then connected to an external suction apparatus connected to a vacuum line (the vacuum line is able to generate a depression of around - 1.0 to -0.7 bar). Firstly, PGSA was applied with the vaccum off over a 1 cm x 1 cm region of the bone and polymerized for 30 seconds with Gecko's proprietary light activation pen (Setalum^{™}). This was performed in triplicate. Then, the same procedure was performed with the vacuum on. Pull of adhesion measurements was then performed using an mechanical testing machine (provided by Instron). The adhesion level of the PGSA to the bone was increased more than 40 times.

### EXAMPLE 4 : PGSA application - submersed artery.

An applicator assembly according to the invention has been used in this study. A dispensing device including a 1 milliliter syringe containing PGSA (Poly(Glycerol) Sebacate Acrylate -US8,143,042) has been connected to an applicator according to figures 8 to 10. The suction tube was connected to an external suction apparatus connected to a vacuum line (the vacuum line is able to generate a depression of around - 0,8 bar). The PGSA was then applied over a porcine carotid artery that was completely submerged in water. When the aspiration was on, the user has applied easily the PGSA on the carotid artery, the aspiration helped stablishing the contact between the delivered PGSA and the other target vessel and though enhancing adhesion. It should be noted, that at the end of the polymer application the vessel was still submersed. Using the same device with the aspiration off, the PGSA deposition was harder and the PGSA had a tendance to do not sit on the vessel surface.

### EXAMPLE 5 : Impact of aspiration on PGSA burst performance.

An applicator assembly according to the invention has been used in this study. A dispensing device including a 1 milliliter syringe containing PGSA (Poly(Glycerol) Sebacate Acrylate -US8,143,042) has been connected to an applicator according to figures 8 to 10. The suction tube was connected to an external suction apparatus connected to a vacuum line (the vacuum line is able to generate a depression of around - 0,8 bar).

A porcine carotid artery was connected to a syringe pump, containing a syringe full of PBS (Phosphate Buffer Saline). On the other extremity of the carotid, a pressure probe was connected to measure liquid pressure inside the carotid artery. After filling the system with PBS, it has been confirmed the system did not present any leak. All the experiment has been performed with the artery wet.

A longitudinal 2-3mm incision was created in carotid artery allow the PBS to leak a environmental pressures.

Firstly, we have applied PGSA with aspiration off over the carotid incision. The maximal burst pressure has been evaluated. After having removed the first layer of polymer, the polymer has been re-applied with the aspiration on. This has been repeated 3 times.

The results show an average increase of PGSA burst pressure of 40% when using aspiration. Moreover, the standard deviation of the values measured was decreased in about 39% Though aspiration improves and standardizes PGSA burst performance.

## Claims

1. An applicator (1) for depositing a layer of adhesive or sealant composition on a target site of a biological and/or prosthetic tissue, the applicator (1) comprising a body (2) having an application face (7) intended to be positioned facing the biological and/or prosthetic tissue, and comprising:
- a first inlet opening (8) for allowing the adhesive or sealant composition to flow from a dispensing device (25) into the applicator (1),
- a first outlet opening (9) provided in the application face (7),
- a first inner channel (10) for guiding the adhesive or sealant composition from the first inlet opening (8) to the first outlet opening (9),
- a second inlet opening (14) provided in the application face (7), adjacent the first outlet opening (9), for allowing pollutants or undesirable elements to be removed from the target site while depositing the layer of adhesive or sealant composition,
- a second outlet opening (15) for allowing the removed pollutants or undesirable elements to be evacuated from the applicator (1),
- a second inner channel (16) for guiding the removed pollutants or undesirable elements from the second inlet opening (14) to the second outlet opening (15),
wherein the first outlet opening (9) has a first section and the second inlet opening (14) has a second section, the second section being greater than the first section,
**characterised by**
the first outlet opening (9) is shaped as a slot for allowing the adhesive or sealant composition to flow out of the applicator (1) and to form the layer of adhesive or sealant composition,
wherein the first outlet opening (9) shaped as a slot has a first width (w₁) and a first height (h₁), and the second inlet opening (14) has a second width (w₂) and a second height (h₂), wherein the second width (w₂) is equal or greater than the first width (w₁).

2. The applicator according to claim 1, wherein:
- the first outlet opening (9) shaped as a slot, has a first width (w₁) which is comprised between 2 and 15 millimeters, preferably comprised between 5 and 8 millimeters, and/or
- the first outlet opening (9) shaped as a slot, has a first height (h₁) which is comprised between 0.2 and 1 millimeters, preferably between 0.4 and 0.6 millimeters, and/or
- the second inlet opening (14) has a second height (h₂) which is comprised between 0.2 and 5 millimeters, preferably between 0.3 and 0.5 millimeters.

3. The applicator according to one of claims 1 to 2, wherein the first inlet opening (8) has a circular shape and the first inner channel (10) has a cross section having a width (w) which continuously increases from the first inlet opening (8) to the first outlet opening (9) and having a height (h) which continuously decreases from the first inlet opening (8) to the first outlet opening (9).

4. The applicator according to one of claims 1 to 3, wherein the body (2) has a first attachment portion (3) adapted for cooperating with an attachment portion (31) of the dispensing device (25) in order to attach the applicator (1) to the dispensing device (25), by translating and/or rotating the first attachment portion (3) of the body (2) relative to the attachment portion (31) of the dispensing device (25) according to a main axis of attachment (X₁).

5. The applicator according to claim 4, wherein the first attachment portion (3) comprises a male conical outer surface adapted to fit with a female conical inner surface of the attachment portion (31) of the dispensing device (25) according to the main axis of attachment (X₁), and lugs (13) protruding at right angle relative to the main axis of attachment (X₁) for locking the first attachment portion (3) of the body (2) to the attachment portion (31) of the dispensing device (25).

6. The applicator according to one of claims 4 and 5, wherein the application face (7) is planar and forms an angle (α) with the main axis of attachment (X₁), the angle being comprised between 90 and 30 degrees, preferably between 90 and 60 degrees, more preferably between 75 and 85 degrees.

7. The applicator according to one of claims 1 to 6, wherein the body (2) comprises a wall (20) separating the first inner channel (16) and the second inner channel (21), the wall (20) having a thickness, measured between the first outlet opening (9) and the second inlet opening (14) which is comprised between 25 micrometers and 1 centimeter.

8. The applicator according to one of claims 1 to 7, wherein the first inner channel (10) has a first main axis (X₁), and the second inner channel (16) has a second main axis (X₂), the second main axis (X₂) being oriented relative to the first main axis (X₁) with an angle (β) comprised between 10 and 20 degrees.

9. The applicator according to one of claims 1 to 8, wherein the body (2) is formed in one single piece of material.

10. The applicator according to claim 9, wherein the material is:
- transparent or translucent to visible light so that an observer can see the adhesive or sealant composition flowing within the first inner channel (21), or
- opaque to radiations having a wavelength comprised between 350 and 670 nanometers, preferably of 405 nanometers.

11. An applicator assembly (44) comprising:
- a dispensing device (25) comprising a reservoir (32) filled with an adhesive or sealant composition, and having an end opening (30) for allowing the adhesive or sealant composition to flow out of the dispensing device (25), and
- an applicator (1) according to claim 1 to 10, adapted to be attached to the dispensing device (25) in order to allow depositing a layer of adhesive or sealant composition flowing from the end opening (30) of the dispensing device (25) on a biological tissue.

12. The applicator assembly according to claim 11, comprising a suction tube (38) and wherein the applicator (1) comprises a second attachment portion (24) for attaching an end (41) of the suction tube (38) to the applicator (1) in order to connect the second inner channel (16) to a suction apparatus.

13. The applicator assembly according to one of claims 11 and 12, wherein the adhesive or sealant composition contained in the reservoir (32) comprises a pre-polymer comprising a polymeric unit of the general formula (-A-B-)ₙ, wherein A represents a substituted or un-substituted ester, B represents a substituted or un-substituted acid ester comprising at least two acid ester functionalities, and n represents an integer greater than 1.

14. The applicator assembly according to one of claims 11 to 13, wherein the dispensing device (25) comprises a syringe (26) having a cylinder (27) defining the reservoir (32), and a piston (28) slidably mounted within the cylinder (27), and comprising a casing (33) adapted to be arranged around the cylinder (27) so as to prevent light from irradiating the adhesive or sealant composition contained within the reservoir (32), wherein the casing (33) comprises resilient tabs (35) which are adapted to be deformed upon insertion of the cylinder (27) within the casing (33) and to abut against a shoulder (36) of the syringe (26) once the cylinder (27) has been inserted in the casing (33), so as to lock the casing (33) on the cylinder (27).

15. The applicator assembly according to one of claims 11 to 14, comprising a packaging (37) for enclosing the applicator (1) and the dispensing device (25), the packaging (37) being made of a material preventing penetration of bacteria.

## Patentansprüche

1. Applikator (1) zum Auftragen einer Schicht einer Kleb- oder Dichtstoffzusammensetzung auf einer Zielstelle eines biologischen und/oder prothetischen Gewebes, wobei der Applikator (1) einen Körper (2) umfasst, der eine Applikationsseite (7) aufweist, die dazu bestimmt ist, dem biologischen und/oder prothetischen Gewebe zugewandt positioniert zu sein, und Folgendes umfasst:
- eine erste Einlassöffnung (8), um es der Kleboder Dichtstoffzusammensetzung zu erlauben, von einer Abgabevorrichtung (25) in den Applikator (1) zu strömen,
- eine erste Auslassöffnung (9), die in der Applikationsseite (7) bereitgestellt ist,
- einen ersten Innenkanal (10) zum Führen der Kleb- oder Dichtstoffzusammensetzung von der ersten Einlassöffnung (8) zur ersten Auslassöffnung (9),
- eine zweite Einlassöffnung (14), die in der Applikationsseite (7) benachbart zu der ersten Auslassöffnung (9) bereitgestellt ist, um beim Auftragen der Schicht von Kleb- oder Dichtstoffzusammensetzung das Beseitigen von Verunreinigungen oder unerwünschten Elementen von der Zielstelle zu erlauben,
- eine zweite Auslassöffnung (15), um das Entleeren der beseitigten Verunreinigungen oder unerwünschten Elemente aus dem Applikator (1) zu erlauben,
- einen zweiten Innenkanal (16) zum Führen der beseitigten Verunreinigungen oder unerwünschten Elemente von der zweiten Einlassöffnung (14) zur zweiten Auslassöffnung (15),
wobei die erste Auslassöffnung (9) einen ersten Querschnitt aufweist und die zweite Einlassöffnung (14) einen zweiten Querschnitt aufweist, wobei der zweite Querschnitt größer als der erste Querschnitt ist,
**dadurch gekennzeichnet, dass**
die erste Auslassöffnung (9) als ein Schlitz geformt ist, um das Ausströmen der Kleb- oder Dichtstoffzusammensetzung aus dem Applikator (1) und das Bilden der Schicht aus Kleb- oder Dichtstoffzusammensetzung zu erlauben,
wobei die erste Auslassöffnung (9), die als ein Schlitz geformt ist, eine erste Breite (w₁) und eine erste Höhe (h₁) aufweist, und die zweite Einlassöffnung (14) eine zweite Breite (w₂) und eine zweite Höhe (h₂) aufweist, wobei die zweite Breite (w₂) gleich oder größer als die erste Breite (w₁) ist.

2. Applikator nach Anspruch 1, wobei:
- die erste Auslassöffnung (9), die als ein Schlitz geformt ist, eine erste Breite (w₁) aufweist, die zwischen 2 und 15 Millimeter, vorzugsweise zwischen 5 und 8 Millimeter, beträgt, und/oder
- die erste Auslassöffnung (9), die als ein Schlitz geformt ist, eine erste Höhe (h₁) aufweist, die zwischen 0,2 und 1 Millimeter, vorzugsweise zwischen 0,4 und 0,6 Millimeter, beträgt, und/oder
- die zweite Einlassöffnung (14) eine zweite Höhe (h₂) aufweist, die zwischen 0,2 und 5 Millimeter, vorzugsweise zwischen 0,3 und 0,5 Millimeter, beträgt.

3. Applikator nach einem der Ansprüche 1 bis 2, wobei die erste Einlassöffnung (8) eine Kreisform aufweist und der erste Innenkanal (10) einen Querschnitt aufweist, der eine Breite (w) aufweist, die von der ersten Einlassöffnung (8) zur ersten Auslassöffnung (9) kontinuierlich zunimmt, und eine Höhe (h) aufweist, die von der ersten Einlassöffnung (8) zur ersten Auslassöffnung (9) kontinuierlich abnimmt.

4. Applikator nach einem der Ansprüche 1 bis 3, wobei der Körper (2) einen ersten Befestigungsabschnitt (3) aufweist, der zum Zusammenwirken mit einem Befestigungsabschnitt (31) der Abgabevorrichtung (25) angepasst ist, um den Applikator (1) durch Translation und/oder Drehung des ersten Befestigungsabschnitts (3) des Körpers (2) in Bezug auf den Befestigungsabschnitt (31) der Abgabevorrichtung (25) gemäß einer Hauptbefestigungsachse (X₁) an der Abgabevorrichtung (25) zu befestigen.

5. Applikator nach Anspruch 4, wobei der erste Befestigungsabschnitt (3) eine aufgenommene konische Außenoberfläche, die angepasst ist, um mit einer aufnehmenden konischen Innenoberfläche des Befestigungsabschnitts (31) der Abgabevorrichtung (25) gemäß der Hauptbefestigungsachse (X₁) zusammenzupassen, und Vorsprünge (13) umfasst, die im rechten Winkel in Bezug auf die Hauptbefestigungsachse (X₁) hervorstehen, um den ersten Befestigungsabschnitt (3) des Körpers (2) an dem Befestigungsabschnitt (31) der Abgabevorrichtung (25) zu verriegeln.

6. Applikator nach einem der Ansprüche 4 und 5, wobei die Applikationsseite (7) eben ist und einen Winkel (α) mit der Hauptbefestigungsachse (X₁) bildet, wobei der Winkel zwischen 90 und 30 Grad, vorzugsweise zischen 90 und 60 Grad, mehr zu bevorzugen zwischen 75 und 85 Grad, beträgt.

7. Applikator nach einem der Ansprüche 1 bis 6, wobei der Körper (2) eine Wand (20) umfasst, die den ersten Innenkanal (16) und den zweiten Innenkanal (21) trennt, wobei die Wand (20) eine zwischen der ersten Auslassöffnung (9) und der zweiten Einlassöffnung (14) gemessene Dicke aufweist, die zwischen 25 Mikrometer und 1 Zentimeter beträgt.

8. Applikator nach einem der Ansprüche 1 bis 7, wobei der erste Innenkanal (10) eine erste Hauptachse (X₁) aufweist, und der zweite Innenkanal (16) eine zweite Hauptachse (X₂) aufweist, wobei die zweite Hauptachse (X₂) in Bezug auf die erste Hauptachse (X₁) mit einem Winkel (β) ausgerichtet ist, der zwischen 10 und 20 Grad beträgt.

9. Applikator nach einem der Ansprüche 1 bis 8, wobei der Körper (2) in einem einteiligen Material gebildet ist.

10. Applikator nach Anspruch 9, wobei das Material Folgendes ist:
- durchsichtig oder durchscheinend gegenüber sichtbarem Licht, derart dass ein Betrachter die innerhalb des ersten Innenkanals (21) strömende Kleboder Dichtstoffzusammensetzung sehen kann, oder
- undurchlässig gegenüber Strahlungen mit einer Wellenlänge von zwischen 350 und 670 Manometern, vorzugsweise von 405 Manometern.

11. Applikatoranordnung (44), die Folgendes umfasst:
- eine Abgabevorrichtung (25), die einen Behälter (32) umfasst, der mit einer Kleb- oder Dichtstoffzusammensetzung gefüllt ist, und eine Endöffnung (30) aufweist, um das Ausströmen der Kleboder Dichtstoffzusammensetzung aus der Abgabevorrichtung (25) zu erlauben, und
- einen Applikator (1) nach Anspruch 1 bis 10, der angepasst ist, um an der Abgabevorrichtung (25) befestigt zu sein, um das Auftragen einer Schicht von Kleb- oder Dichtstoffzusammensetzung, die aus der Endöffnung (30) der Abgabevorrichtung (25) strömt, auf ein biologisches Gewebe zu erlauben.

12. Applikatoranordnung nach Anspruch 11, die ein Saugrohr (38) umfasst, und wobei der Applikator (1) einen zweiten Befestigungsabschnitt (24) zum Befestigen eines Endes (41) des Saugrohrs (38) an dem Applikator (1) umfasst, um den zweiten Innenkanal (16) mit einer Saugvorrichtung zu verbinden.

13. Applikatoranordnung nach einem der Ansprüche 11 und 12, wobei die Kleb- oder Dichtstoffzusammensetzung, die in dem Behälter (32) enthalten ist, ein Vorpolymer umfasst, das eine Polymereinheit mit der allgemeinen Formel (-A-B-)ₙ umfasst, wobei A einen substituierten oder unsubstituierten Ester darstellt, B einen substituierten oder unsubstituierten Säureester darstellt, der mindestens zwei Säureesterfunktionalitäten umfasst, und n eine Ganzzahl größer als 1 darstellt.

14. Applikatoranordnung nach einem der Ansprüche 11 bis 13, wobei die Abgabevorrichtung (25) eine Spritze (26), die einen Zylinder (27) aufweist, der den Behälter (32) definiert, und einen Kolben (28) umfasst, der innerhalb des Zylinders (27) verschiebbar gelagert ist, und ein Gehäuse (33) umfasst, das angepasst ist, um um den Zylinder (27) angeordnet zu sein, um zu verhindern, dass Licht die Kleb- oder Dichtstoffzusammensetzung, die innerhalb des Behälters (32) enthalten ist, bestrahlt, wobei das Gehäuse (33) elastische Laschen (35) umfasst, die angepasst sind, um bei der Einsetzung des Zylinders (27) innerhalb des Gehäuses (33) verformt zu werden und um gegen eine Schulter (36) der Spritze (26) anzustoßen, nachdem der Zylinder (27) in das Gehäuse (33) eingesetzt wurde, um das Gehäuse (33) an dem Zylinder (27) zu verriegeln.

15. Applikatoranordnung nach einem der Ansprüche 11 bis 14, die eine Verpackung (37) zum Umschließen des Applikators (1) und der Abgabevorrichtung (25) umfasst, wobei die Verpackung (37) aus einem Material besteht, welches das Eindringen von Bakterien verhindert.

## Revendications

1. Applicateur (1) pour déposer une couche de composition adhésive ou d'étanchéité sur un site cible d'un tissu biologique et/ou prothétique, l'applicateur (1) comprenant un corps (2) ayant une face d'application (7) destinée à être positionnée face au tissu biologique et/ou prothétique, et comprenant :
- une première ouverture d'entrée (8) pour permettre à la composition adhésive ou d'étanchéité de s'écouler d'un dispositif de distribution (25) dans l'applicateur (1),
- une première ouverture de sortie (9) prévue dans la face d'application (7),
- un premier canal intérieur (10) pour guider la composition adhésive ou d'étanchéité depuis la première ouverture d'entrée (8) jusqu'à la première ouverture de sortie (9),
- une deuxième ouverture d'entrée (14) prévue dans la face d'application (7), adjacente à la première ouverture de sortie (9), pour permettre aux polluants ou aux éléments indésirables d'être éliminés du site cible pendant le dépôt de la couche de composition adhésive ou d'étanchéité,
- une deuxième ouverture de sortie (15) pour permettre aux polluants ou éléments indésirables retirés d'être évacués de l'applicateur (1),
- un second canal intérieur (16) pour guider les polluants ou les éléments indésirables retirés de la seconde ouverture d'entrée (14) vers la seconde ouverture de sortie (15),
dans lequel la première ouverture de sortie (9) a une première section et la deuxième ouverture d'entrée (14) a une deuxième section, la deuxième section étant supérieure à la première section,
**caractérisé en ce que**
la première ouverture de sortie (9) a la forme d'une fente permettant à la composition adhésive ou d'étanchéité de s'écouler hors de l'applicateur et de former la couche d'adhésif ou de composition d'étanchéité, la première ouverture de sortie (9) est en forme de fente pour permettre à lacomposition d'adhésif ou de mastic,
dans laquelle la première ouverture de sortie (9) en forme de fente a une première largeur (w1) et une première hauteur (h1), et la deuxième ouverture d'entrée (14) a une deuxième largeur (w2) et une deuxième hauteur (h2), la deuxième largeur (w2) étant égale ou supérieure à la première largeur (w1).

2. L'applicateur selon la revendication 1, dans lequel :
- la première ouverture de sortie (9) en forme de fente, a une première largeur (w1) comprise entre 2 et 15 millimètres, de préférence comprise entre 5 et 8 millimètres, et/ou
- la première ouverture de sortie (9) en forme de fente, a une première hauteur (h1) qui est comprise entre 0,2 et 1 millimètre, de préférence entre 0,4 et 0,6 millimètre, et/ou
- la deuxième ouverture d'entrée (14) a une deuxième hauteur (h2) qui est comprise entre 0,2 et 5 millimètres, de préférence entre 0,3 et 0,5 millimètres.

3. L'applicateur selon l'une des revendications 1 à 2, dans lequel la première ouverture d'entrée (8) a une forme circulaire et le premier canal intérieur (10) a une section transversale ayant une largeur (w) qui augmente continuellement de la première ouverture d'entrée (8) à la première ouverture de sortie (9) et ayant une hauteur (h) qui diminue continuellement de la première ouverture d'entrée (8) à la première ouverture de sortie (9).

4. L'applicateur selon l'une des revendications 1 à 3, dans lequel le corps (2) possède une première partie de fixation (3) adaptée pour coopérer avec une partie de fixation (31) du dispositif de distribution (25) afin de fixer l'applicateur (1) au dispositif de distribution (25), par la translation et/ou la rotation de la première partie de fixation (3) du corps (2) par rapport à la partie de fixation (31) du dispositif de distribution (25) selon un axe principal de fixation (X1).

5. L'applicateur selon la revendication 4, dans lequel la première partie de fixation (3) comprend une surface extérieure conique mâle adaptée pour s'adapter à une surface intérieure conique femelle de la partie de fixation (31) du dispositif de distribution (25) selon l'axe principal de fixation (X1), et des pattes (13) faisant saillie à angle droit par rapport à l'axe principal de fixation (X1) pour verrouiller la première partie de fixation (3) du corps (2) à la partie de fixation (31) du dispositif de distribution (25).

6. L'applicateur selon l'une des revendications 4 et 5, dans lequel la face d'application (7) est plane et forme un angle (α) avec l'axe principal de fixation(X1), l'angle étant compris entre 90 et 30 degrés, de préférence entre 90 et 60 degrés, de préférence entre 75 et 85 degrés.

7. L'applicateur selon l'une des revendications 1 à 6, dans lequel le corps (2) comprend une paroi (20) séparant le premier canal intérieur (16) et le second canal intérieur (21), la paroi (20) ayant une épaisseur, mesurée entre la première ouverture de sortie (9) et la seconde ouverture d'entrée (14), comprise entre 25 micromètres et 1 centimètre.

8. L'applicateur selon l'une des revendications 1 à 7, dans lequel le premier canal interne (X1), et le second canal intérieur (16) a un second axe principal (X2), le second axe principal (X2) étant orienté par rapport au premier axe principal (X1) avec un angle ([3) compris entre 10 et 20 degrés.

9. L'applicateur selon l'une des revendications 1 à 8, dans lequel le corps (2) est formé d'une seule pièce de matériau.

10. L'applicateur selon la revendication 9, dans lequel le matériau est :
- transparent ou translucide à la lumière visible de sorte qu'un observateur puisse voir la composition adhésive ou de scellement s'écouler dans le premier canal intérieur (21), ou
- opaque aux radiations ayant une longueur d'onde comprise entre 350 et 670 nanomètres, de préférence 405 nanomètres.

11. Ensemble applicateur (44) comprenant :
- un dispositif de distribution (25) comprenant un récipient (32) rempli d'une composition adhésive ou d'étanchéité et ayant une ouverture d'extrémité (30) pour permettre à la composition adhésive ou d'étanchéité de s'écouler du dispositif de distribution (25), et
- un applicateur (1) selon les revendications 1 à 10, adapté pour être fixé au dispositif de distribution (25) pour permettre l'application sur un tissu biologique d'une couche de composition adhésive ou d'étanchéité s'écoulant de l'ouverture d'extrémité (30) du dispositif de distribution (25).

12. L'ensemble applicateur selon la revendication 11, comprenant un tube d'aspiration (38) et dans lequel l'applicateur (1) comprend une deuxième partie de fixation (24) pour fixer une extrémité (41) du tube d'aspiration (38) à l'applicateur (1) afin de connecter le deuxième canal intérieur (16) à un appareil d'aspiration.

13. Ensemble applicateur selon l'une des revendications 11 et 12, dans lequel la composition adhésive ou de scellement contenue dans le réservoir (32) comprend un pré-polymère comprenant une unité polymérique de formule générale (-A-B-)ₙ, dans laquelle A représente un ester substitué ou non substitué, B représente un ester acide substitué ou non substitué comprenant au moins deux fonctionnalités d'ester acide, et n représente un nombre entier supérieur à 1.

14. L'ensemble applicateur selon l'une des revendications 11 à 13, dans lequel le dispositif de distribution (25) comprend une seringue (26) ayant un cylindre (27) définissant le réservoir (32), et un piston (28) monté de manière coulissante à l'intérieur du cylindre (27), et comprenant un boîtier (33) adapté pour être disposé autour du cylindre (27) de manière à empêcher la lumière d'irradier la composition adhésive ou de scellant contenue dans le réservoir (32), dans lequel le boîtier (33) comprend des languettes élastiques (35) qui sont adaptées pour être déformées lors de l'insertion du cylindre (27) dans le boîtier (33) et pour s'appuyer contre un épaulement (36) de la seringue (26) une fois que le cylindre (27) a été inséré dans le boîtier (33), de manière à verrouiller le boîtier (33) sur le cylindre (27).

15. L'ensemble applicateur selon l'une des revendications 11 à 14, comprenant un emballage (37) pour contenir l'applicateur (1) et le dispositif de distribution (25), l'emballage (37) étant constitué d'un matériau empêchant la pénétration des bactéries.
